# EUROPÄISCHE PATENTSCHRIFT

(11) **EP 0 668 246 B1**
(45) Veröffentlichungstag und Bekanntmachung des Hinweises auf die Patenterteilung: **14.06.2000**
(21) Anmeldenummer: 93908187.3
(22) Anmeldetag: 15.02.1993
(51) Int. Cl.: C02F 3/34, C12N 1/20

(54) **Verfahren unter Anwendung der Stämme von Acinetobacter Species (Bicoccum), Arthrobacter Species und Rhodococcus Species zur biologischen Behandlung von Ölverunreinigungen**
Process using the strains of Acinetobacter species (Bicoccum), Arthrobacter species and Rhodococcus species for the biological treatment of oil pollution
Procédé de traitement biologique de produits petroliers polluants en utilisant des souches d'espece Acinetobacter (Bicoccum), Arthrobacter et Rhodococcus

(43) Veröffentlichungstag der Anmeldung: 23.08.1995
(73) Patentinhaber: Murzakov, Boris Gerasimovich, 142712 Moskovskaya obl. (RU)
(72) Erfinder: MURZAKOV, Boris Gerasimovich, Gorki Leninskie, 142712 (RU); ZAIKINA, Alexandra Ivanovna, Moscow, 117437 (RU); ROGACHEVA, Rufina Alexandrovna, Moscow, 114461 (RU); SEMENOVA, Elena Vladimirovna, Vidnoe, 142711, Moskovskaya obl. (RU)
(74) Vertreter: von Füner, Alexander, Prof.h.c. Dr.
(86) Internationale Anmeldenummer: RU9300045
(87) Internationale Veröffentlichungsnummer: WO9418132

(56) Entgegenhaltungen:
- EP-A- 0 141 784
- GB-A- 2 252 342
- SU-A- 612 958
- SU-A- 823 314
- US-A- 3 069 325
- US-A- 3 871 957
- US-A- 3 941 692
- US-A- 4 605 502
- US-A- 4 808 535

## Beschreibung

Die vorliegende Erfindung bezieht sich auf die Mikrobiologie, insbesondere auf ein Verfahren zur biologischen Reinigung von Ölverschmutzungen unter Benutzung von Stämmen von Acinetobacter species (bicoccum), Arthrobacter species und/oder Rhodococcus species.

Die genannten Stämme können breite Verwendung finden bei der Regenerierung der Ökologie der Umwelt, in Regionen der Gewinnung und Nutzung von Erdöl und Erdölprodukten sowie zur Rekultivierung des Bodens und Reinigung von Gewässern.

Ökologische Störungen der umgebenden Natur, hervorgerufen durch ausgeflossenes Öl in Gebieten seiner Gewinnung, Speicherung, Fortleitung, Verarbeitung und Nutzung, erfahren eine immer stärkere Verbreitung. Nach Angaben der Weltliteratur werden jährlich allein durch Havarien von Tankern bis zu 10 Mill. t Erdöl verkippt. Bei Ölhavarien kommt es zum Tod lebender Organismen, die das Ökosystem bilden, und zur Wiederherstellung seiner Ganzheit ist eine Initiierung des Abbauprozesses von Kohlenwasserstoffen erforderlich. In Gebieten der Erdölgewinnung werden Tausende von Hektar fruchtbarer Böden und Weiden der landwirtschaftlichen Nutzung entzogen, Fischteichs und Trinkwasserquellen verschmutzt. Ein großes Problem ist auch die Reinigung von Behältern und Tankern von Erdölprodukten.

Unter Naturbedingungen sind zahlreiche und vielfältige Gruppen von Mikroorganismen anwesend, die Erdölkohlenwasserstoffe verbrauchen. Jedoch ist die Fähigkeit dieser Mikroorganismen in bezug auf Zeit und Reinigungsgrad völlig unbefriedigend, besonders bei einer intensiven Verschmutzung von Böden und Gewässern.

Es gibt verschiedene Verfahren zur Reinigung von Ölverschmutzungen. Einen besonderen Platz behalten biologische Reinigungsverfahren. Solche Verfahren sehen den Einsatz von Mikroorganismen, Bakterien, Hefen, Pilzen vor.

Bekannt ist die Benutzung des Naturstammes Pseudomonas putida-36 in einem Verfahren zur Reinigung des Bodens von Ölverschmutzungen (SU, A, 1428809). Nach diesem Verfahren nimmt man die Reinigung durch Einführen der Biomasse der Bakterienkultur des genannten Stammes in die Ölverschmutzung vor. Die Bakterienkultur bringt man gemeinsam mit einem Mineraldünger ein, der Stickstoff in Nitratform enthält. Die Menge der einzubringenden Kultur beträgt nicht unter 10⁴ Zellen in 1 mg/l bei einem Verbrauch des wässerigen Gemisches von 0,5 bis 1 l/m².

Das Verfahren erlaubt es, den Boden von Ölverschmutzungen zu reinigen. Jedoch züchtet man die verwendete Bakterienkultur auf einem defizitären Nahrungs-Kohlenhydratsubstrat. Das gewählte Substrat ist nicht optimal, weil an seiner Oxidation Fermente beteiligt sind, die von den Fermenten des Abbaus von Erdöl verschieden sind. Dies führt zu einer Senkung der Reinigungsintensität wegen der Adaption der Kultur an das neue Substrat. Außerdem besitzt der besagte Stamm Pseudomonas putida-36 eine hämolytische und gelatinöse Aktivität, was die Entwicklung des Prozesses der Selbstreinigung mit Hilfe von Mikroorganismen behindert.

Bei der Durchführung dieses Verfahrens ist der erreichbare Reinigungsgrad unzureichend hoch. Das Verfahren hat eine beschränkte Anwendung. Praktisch eignet es sich nur zur Reinigung des Bodens. Darüberhinaus führt die Anwendung der Nitratformen von Stickstoff bei der Durchführung dieses Verfahrens zu einer zusätzlichen Verschmutzung der Umgebung mit toxischen Nitrationen.

Die GB 2 252 342 offenbart ein Verfahren zur mikrobiellen Gewinnung von Öl aus einer Erdöl führenden Gesteinsformation, die eine Zuflußleitung an einer ersten Stelle und eine Abflußleitung an einer zweiten Stelle einschließt, das folgende Schritte umfaßt:
- Einspritzen von Wasser, das eine Sauerstoffquelle, die mindestens 5 mg/l freien Sauerstoff liefern kann, enthält, in die Formation an der ersten Stelle, die im Abstand von der zweiten Stelle angeordnet ist,
- Vermehrung der Mikroorganismen, die entweder bereits in der Formation vorhanden sind oder gleichzeitig mit dem Sauerstoff enthaltenden Einspritzwasser eingeführt werden, wobei die Mikroorganismen das Öl als hauptsächliche Kohlenstoffquelle und den Sauerstoff aus dem Einspritzwasser als hauptsächliche Sauerstoffquelle verwenden, wodurch eine Schicht aus einer Biomasse gebildet wird, die dazu dient, das Öl von der Gesteinsformation zu trennen; und
- Entfernung des abgetrennten Öls mit Hilfe des Einspritzwassers über die Abflußleitung.

Als Mikroorganismen können gemäß GB 2 252 342 u.a. solche der Gattung Acinetobacter verwendet werden.

Die US 3 941 692 offenbart ein Verfahren zum Entfernen von Öl aus Seewasser in Schiffsfrachträumen, das folgende Schritte umfaßt:
- Zugabe eines Mikroorganismus, einer Stickstoffquelle und einer Phosphorquelle zu Öl enthaltendem Seewasser in einem Schiffsfrachtraum, und
- Umwandlung des erhaltenen Gemisches mit Hilfe dieses Mikroorganismus oder den Produkten dieses Mikroorganismus unter aeroben Bedingungen in diesem Schiffsfrachtraum in eine nichtölige Form, wobei dieser Mikroorganismus der Gattung Arthrobacter angehört und als RAG-1 (ATCC Nr. 31012) bezeichnet wird.

Die US 3 871 957 offenbart ein Verfahren, um die feste Oberfläche von Stränden und Küstenlinien gegen Ölverschmutzung zu schützen, das folgende Schritte umfaßt:
- Vorbehandlung der festen Oberfläche eines Strandes oder einer Küstenlinie mit einer wässerigen Aufschlämmung, die hergestellt wird durch Beimischung von Zellen von miteinander verträglichen Mikroorganismen, die ausgewählt werden aus der Gruppe bestehend aus Arthrobacter, Mikrococcus, Achromobacter und deren Gemischen, zu einem inerten absorbierenden Stoff;
- Trocknung dieses Gemisches bei einer Temperatur zwischen 25° und 55°C, wobei ein wasserfreies Pulver erhalten wird;
- erneutes Auflösen dieses wasserfreien Pulvers, wobei eine Aufschlämmung erhalten wird, wodurch die Lebensfähigkeit und biologische Aktivität der Mikroorganismen erhalten wird, und
- Beschichtung der festen Oberfläche des Strandes oder der Küstenlinien mit dieser wässerigen Aufschlämmung von lebensfähigen, miteinander verträglichen Mikroorganismuszellen. Zur Verwendung in diesem Verfahren wird aus der Gattung Arthrobacter die Art Arthrobacter Nov. sp. (ATCC Nr. 21908) vorgeschlagen.

Der Erfindung liegt die Aufgabe zugrunde, durch Selektion und Verwendung neuer Stämme ein biologisches Verfahren zur Reinigung von Ölverschmutzungen bereitzustellen, das es gestattet, verschiedene verschmutzte Objekte mit großer Intensität und hohem Reinigungsgrad zu reinigen, ohne die Ökologie der Umwelt zu stören.

Diese Aufgabe ist wie aus Anspruch 1 ersichtlich gelöst worden.

Die erfindungsgemäß verwendeten Stämme Acinetobacter species (bicoccum) B-6445, Arthrobacter species S-1212 und Rhodococcus species S-1213 sind saprophitische Naturstämme. Sie besitzen eine hohe Oxidationsfähigkeit gegenüber einem breiten Spektrum von Kohlenwasserstoffen unterschiedlichen chemischen Aufbaus und sind durch die Fähigkeit einer schnellen Akklimatisierung in Naturzönosen gekennzeichnet.

Die Stämme assimilieren als die einzige Kohlenhydratquelle ein breites Spektrum von zur Zusammensetzung von Erdöl gehörenden Kohlenwasserstoffen: n-Alkane C₈-C₃₈, Cycloalkane, aromatische Kohlenwasserstoffe, Asphaltene, ungereinigte Flüssigkeitsgemische von Paraffinen, barzige Stoffe, Öle auf Erdölbasis, Dieseifraktionen, Masut und Rohöl (insgesamt über 25 Rohölbestandteile ).

Im Prozess der Lebenstätigkeit von Mikroorganismen verwandeln sich die von diesen Stämmen ausgenutzten Kohlenwasserstoffe in zellulare Stoffe (Eiweissstoffe, Lipide, Nukleinsäuren, Kohlenhydrate) und Kohlendioxidgas, welche dann in den Gesamtzyklus des Kreislaufs von Stoffen in der Biosphäre einbezogen werden. Nach Abschluss der Reinigung von Erdölprodukten wird die gebildete Biomasse unter der Einwirkung trophischer Regulationsmechanismen in den Zyklus natürlicher Mikrobiozönosen einbezogen, und die überschüssige Menge derselben stirbt ab, indem sie sich unter Einwirkung der saprophitischen Mikroflora in Humusstoffe des Bodens oder in eine vollwertige Eiweiss-Vitamin-Masse, die von der Wasserfauna verbraucht wird, verwandelt.

Die Stämme sind nichtpathogen, nichttoxisch und weisen keine hämolytische Aktivität auf.

Der Stamm Acinetobacter species (bicoccum) B-6445 ist aus aktiviertem Schlamm im Durchfluss selektioniert, ist gramnegativ und durch folgende kulturmorphologische und physiologisch-biochemische Merkmale gekennzeichnet.

### Kulturmorphologische Merkmale

Zellen auf dem Fleisch-Pepton-Agar sind unbeweglich. Sie stellen in der jungen Kultur kurze und dicke Stäbchen mit einer Grösse von 1,2-1,8·1,5-3,4 µm dar, in der dreitätigen Kultur haben sie Kokkenform mit 1,5 µm Grösse. Die Kultur bildet keine Sporen. Kolonien auf dem Fleisch-Pepton-Agar (dreitätige Kultur) sind rundlieh mit ebenem Rand, weisslich, glänzend, die Konsistenz ist sauerrahmartig.

### Physiologisch-biochemische Merkmale.

Obligater Aerobier. Chemoorganotroph. Katalasepositiv. Oxydasenegativ. Ein Bedarf an Vitaminen und zusätzlichen Wachstumsfaktoren fehlt. Wächst in weitem Bereich des pH-Wertes des Mediums von 3,0-9,0 und der Temperaturen von 10-45°C. Optimale Wachstumstemperatur 35-40°C. Er benutzt Mineralstickstoff.

Der Stamm Arthrobacter species S-1212 ist aus aktiviertem Schlamm im Durchfluss selektioniert, ist gramvariabel und durch folgende kulturmorphologische und physiologisch-biochemische Merkmale gekennzeichnet.

### Kulturmorphologische Merkmale.

Zeilen auf dem Fleisch-Pepton-Agar sind unbeweglich und stellen grampositive Kokken und gramnegative Stäbchen dar. Die Zeilen der dreitätigen Kultur von stäbchenartiger unregelmässiger Gestalt variieren nach Grösse und Form, liegen oft unter einem Winkel zueinander, indem sie V-Formen bilden. Kolonien auf dem Fleisch-Pepton-Agar (dreitätige Kultur) sind rundlich mit ebenem Rand, weisslich mit Glanz, gleichmässig konvex. Die Kultur bildet keine Sporen.

### Physiologisch-biochemische Merkmale.

Obligater Aerobier. Chemoorganotroph. Katalasepositiv. Oxydasenegativ. Ein Bedarf an Vitaminen und zusätzlichen Wachstumsfaktoren fehlt. Wächst in weitem Bereich des pH-Wertes des Mediums von 3,0-8,5 und der Temperaturen von 10-35°C. Er benutzt Mineralstickstoff. Bindet Luftstickstoff.

Der Stamm Rhodococcus species S-1213 ist aus aktiviertem Schlamm im Durchfluss selektioniert, ist grampositiv und durch folgende kulturmorphologische und physiologisch-biochemische Merkmale gekennzeichnet.

### Kulturmorphologische Merkmale.

Zellen auf dem Fleisch-Pepton-Agar sind unbeweglich, vereinzelt von kurzovaler Form und Stäbchen, die nach Form und Grösse variieren, Die Zellen in der jungen Kultur können Ketten und in der alten Kultur Kokken bilden. Die Grösse der Stäbchen 1,0·1,7 µm. Die Kultur bildet keine Sporen.

Kolonien auf dem Fleisch-Pepton-Agar (dreitätige Kultur) sind rundlich mit ebenem Rand, hellcremefarben mit Glanz, gleichmässig erhaben.

### Physiologisch-biochemische Merkmale.

Obligater Aerobier. Chemoorganotroph. Katalasepositiv, oxydasenegativ. Ein Bedarf an Vitaminen und zusätzlichen Wachstumsfaktoren fehlt. Wächst in weitem Bereich des pH-Wertes des Mediums von 3,0-8,5 und der Temperaturen von 10-43°C. Optimale Temperatur 25-30°C. Er benutzt Mineralstickstoff.

Die Biomasse der Bakterienkultur gewinnt man auf herkömmlichem Wege durch Kultivierung der Stämme Acinetobacter species (bicoccum) B-6445, Arthrobacter species S-1212, Rhodococcus species S-1213 auf einem beliebigen Stndardnährmedium, beispielsweise auf dem mineralischen Medium, das Quellen von Stickstoff und Phosphor und Spurenelemente enthält. Als solches Medium kann das Hottinger-Medium, King-Medium dienen, wo als Kohlenstoffquelle Kohlenhydrate, organische Säuren, Alkohole, Kohlenwasserstoffe, Paraffine oder Rohöl in Frage kommen können. Die Bedingungen der Bakterienzüchtung sind ebenfalls herkömmlich.

Bei dem erfindungsgemäßen Verfahren zur biologischen Reinigung von Ölverschmutzungen, welches das Einbringen einer Bakterienkultur in die Verschmutzung einschließt, bringt man als Bakterienkultur den Stamm Acinetobacter species (bicoccum) B-6445, Arthrobacter species S-1212 und/oder Rhodococcus species S-1213, selektioniert aus aktiviertem Schlamm und hinterlegt am 19. Januar 1993 in der Unionskollektion industrieller Mikroorganismen des Unions-Forschungsinstituts für Genetik und Selektion von Mikroorganismen, in die Verschmutzung ein, wobei das Masseverhältnis der Biomasse der Bakterienkultur zur Ölverschmutzung jeweils 1:10 - 10⁵ beträgt.

Wie vorstehend angegeben, kennzeichnen sich die erfindungsgemäß verwendeten Stämme durch eine hohe Wachstumsaktivität auf dem mineralischen Medium, in dem als einzige Kohlenstoffquelle Rohöl und Erdölprodukte enthalten sind. Beim Einbringen der Biomasse dieser Bakterienkulturen in die Ölverschmutzung wird das Substrat von Bakterien verbraucht, was zum Abbau von Erdölprodukten führt, wodurch eben die Reinigung der Ölverschmutzung sichergestellt wird.

Das vorerwähnte Massenverhältnis der Biomasse der Bakterienkultur zur Ölverschmutzung ist optimal. Es gewährleistet die Reinigung mit einem hoben Reinigungsgrad (90 - 100%). Beim Einoringen der Biomasse der Bakterienkultur in das verschmutzte Objekt in einer Menge kleiner als im angegebenen Verhältnis nimmt die Reinigungsdauer erheblich zu, während bei einer Vergrösserung des Verbrauchs der Biomasse der Bakterienkultur über den genannten oberen Grenzwert binaus die Reinigungswirkung nicht steigt, die Aufwendungen für die Reinigung aber grösser werden.

In manchen Fällen, wenn in dem zu reinigenden Objekt Stickstoff- und Phosphorsalze fehlen oder in einer geringen Menge vorhanden sind, empfiehlt es sich, die biologische Reinigung in Anwesenheit der Mineralsalze von Stickstoff und Phosphor durchzuführen.

Zwecks Intensivierung des Reinigungsprozesses empfiehlt es sich auch, die Reinigung in Anwesenheit von Spurenelementen, nämlich Eisen, Zink, Mangan und/oder Magnesium, durchzuführen. Das Vorhandensein dieser Elemente begünstigt den Abbau des Erdöls.

Die Menge von Mineralstoffzusatzmitteln (Quellen von Stickstoff, Phosphor, Kalium und Magnesium usw.) hängt von der Zusammensetzung des zu reinigenden Objektes (des Bodens, des Wassers) ab und wird aufgrund der Bestimmung der Konzentration dieser Elemente im Naturobjekt und der Errechnung ihrer mangelnden Menge, die zum Abbau der Ölverschmutzung notwendig ist, berechnet.

Das erfindungsgemässe Verfahren zur biologischen Reinigung von Ölverschmutzungen gestattet es, verschmutzte Objekte unabhängig von dem Verschmutzungsgrad mit einer hohen Effektivität zu reinigen. Der Reinigungsgrad beträgt bei der Durchführung des erfindungsgemäss vorgeschlagenen Verfahrens etwa 100%. Das Verfahren ist universal. Es eignet sich zur Reinigung von Böden, natürlichen Gewässern und Kunstbecken, industriellen Abwässern sowie verschiedenen technologischen Ausrüstungen. Das Verfahren ist umweltfreundlich. Die eingebrachte Biomasse der Bakterienkultur erfordert keine Anwendung irgendwelcher zusätzlichen Stoffe. Im Falle der Benutzung von Mineralsalzen werden die letzteren im Prozess des Metabolismus der Bakterien vollständig verbraucht. Die eingentliche Biomasse der Bakterienkultur wird in die natürliche Biozönose des Bodens einbezogen, indem sie dessen agrotechnischen Zustand verbessert, oder sie dient als zusätzliche Quelle der Planktonnahrung im Falle der Reinigung von Gewässern. Ausgeschlossen wird auch die Verunreinigung von Grundwässern, weil die erfindungsgemäss vorgeschlagenen Naturstämme obligate Aerobier sind, die in Abwesenheit von Sauerstoff verkümmern. Das Verfahren erlaubt es, den Prozess der Rekultivierung von landwirtschaftlichen Böden und die Reinigung von Fischteichen zu intensivieren, das heisst die Rückführung von Böden und Gewässern in die volkswirtschaftliche Nutzung unter gleichzeitiger "Gesundung" der Umwelt zu verwirklichen.

Das Verfahren ist auch durch einen geringen Verbrauch an Biomasse gekennzeichnet.

Das Verfahren zur biologischen Reinigung von Ölverschmutzungen ist einfach in der technologischen Ausführung und wird folgendermassen durchgeführt.

In Abhängigkeit von der Zweckbestimmung verwendet man die gewonnene Biomasse der Bakterienkultur in Form einer wässrigen Suspension, einer Paste oder eines Pulvers. So wird z.B. die Biomasse bei der Reinigung von Gewässern zweckmässig in Form eines Pulvers und bei der Reinigung des Bodens in Form einer Suspension oder einer Paste eingesetzt.

Die Suspension und die Paste verwendet man in der Regel in Gebieten, die an die Betriebe für ihre Produktion unmittelbar angenähert sind. Die Haltbarkeitsdauer einer solchen Biomasse beträgt (bei einer Temperatur unter 15°C) bis zu 8 Monaten. Die Biomasse im ausgetrockneten Zustand hat eine Haltbarkeitsdauer von mindestens 2 Jahren. Sie stellt ein feindisperses leicht krümeliges Pulver von gelblich-grauer Farbe dar und hält sich gut in Kraftpapierbeuteln an einer trockenen Stelle bei erniedrigter (aber nicht negativer) Temperatur.

Bei Verwendung von Mineralstoffzusatzmitteln führt man diese entweder im Gemisch mit der Biomasse der Bakterienkultur oder gesondert in Form von wässrigen Lösungen ein.

Die Bearbeitung der verschmutzten Abschnitte von Boden, Wasser, Behältern und anderen Objekten erfolgt mit Hilfe verschiedener technologischer Maschinen, Aggregate, Feuerlöschzüge, Hubschrauber oder Flugzeuge sowie such von Hand.

Das erfindungsgemässe Verfahren sieht die Bodenbearbeitung ohne Abheben der Schichten des Bodens vor, obwohl in einigen Fällen dessen Auflockerung durchgeführt werden muss (verschmutzter Sand, Kies, von Erdöl durchtränkter Boden).

Die Reinigung von Gewässern setzt ein Auftragen der Biomasse der Bakterienkultur auf die verschmutzte Oberfläche beispielsweise durch Zerstäubung voraus.

Die Reinigung von Behältern geschieht durch Eintragen einer im voraus zubereiteten Suspension mit nachfolgender Vermischung derselben in die Behälter.

Zum besseren Verständnis der vorliegenden Erfindung werden folgende konkrete Beispiele angeführt.

### Beispiel 1

In einen Behälter mit 150 cm Höhe und 50 cm Durchmesser füllt man 200 l Wasser ein, das 4 Masse-% Seesalz enthält und auf dessen Oberfläche sich 1 kg Rohöl mit 5 mm Schichtdicke befindet. In den Behälter gibt man 20 g Biomasse der Bakterienkultur des Stammes Rhodococcus species S 1213 in Pulverform zu (Massenverhältnis der Biomasse zum Erdöl 1:10²). Den Inhalt des Behälters verrührt man durch Barbotage mittels Luft. Nach 2 Tagen hat sich der Gehalt an n-Alkanen um 92,4% und an aromatischen Kohlenwasserstoffen um 90,1% verringert. Der Prozess der vollständigen Reinigung (100%) ist im Laufe von 7 Tagen abgeschlossen.

### Beispiel 2

Ein 200 Liter fassendes Gefäss ist am Boden und an den Wänden mit 3 kg Erdölprodukten (Masut) verschmutzt. Man nimmt ein mechanisches Waschen des Gefässes mit Wasser (100 l) vor, dem der Stamm Acinetobacter species (bicoccum) B-6445 in Form einer Suspension mit der Konzentration 0,1 g absolut trockene Biomasse/l (Massenverhältnis der Biomasse zum Masut 1:10³) zugesetzt ist. In das Gefäss gibt man auch folgende Mineralsalze (g/l) zu: NH₄H₂PO₄-10; KH₂PO₄-10; MgSO₄ 7H₂0-0,7. Nach 2 Tagen hat sich der Gehalt an n-Alkanen um 93,7% und an aromatischen Kohlenwasserstoffen um 89,6% verringert. Die vollständige Reinigung (100%) ist nach 8 Tagen abgeschlossen.

### Beispiel 3

Ein Bodenabschnitt mit 1 ha Fläche ist mit Rohöl in einer Menge von 12,5 kg/m² (12,5 t) verschmutzt. Wach einmaliger Behandlung mit der Biomasse der Bakterienkultur in Form einer wässrigen Suspension des Stammes Arthrobacter species S-1212 in einer Menge von 12 kg absolut trockener Biomasse (Massenverhältnis der Biomasse zum Erdöl 1:10³) werden nach 7 Tagen auf der Bodenoberfläche folgende Änderungen visuell beobachtet; die ölige Oberfläche wird matt, mit kleinen Poren durch Ausscheidung von Kohlendioxidgas durchsetzt, die Farbe verändert sich von der schwarzen bis zur grauen und dann dunkelgelben. Die Zusammensetzung der Bodenaggregate wird aus einer zähen schlammigen Konsistenz krümelig mit charakteristischem feuligem Geruch. Nach 14 Tagen sind die Bodenkrumen nicht brennbar, nach 21 Tagen werden die Erdölprodukte bei der chemischen Analyse nicht nachgewiesen, die Zusammensetzung dar Mikroflora wird wiederhergestellt. Der Reinigungsgrad beträgt praktisch 100%.

### Beispiel 4

Das Becken eines Erdöltanklagers mit 1 ha Fläche enthält 183 t Rohöl. Nach einmaliger Behandlung des Beckens mit einer Suspension der Biomasse der Bakterienkultur des Stammes Arthrobacter species S-1212, die 12 kg absulut trockene Biomasse enthält (Massenverhältnis der Biomasse zum Erdöl 1:10⁵), treten nach 7 Tagen auf der Oberfläche Aufblähungen auf, die durch Ausscheidung von Kohlendioxidgas bedingt sind. Der Ölfilm wird aufeinanderfolgend braun, dunkelrot, hellorange und zerfällt in schnell verschwindende Fragmente, die mit weisslichen Ansammlungen der Mikrobenmasse durchsetzt sind. Nach 20 Tagen verschwindet der Film (100%-ige Reinigung) die Durchsichtigkeit des Wassers ist nach 30 Tagen völlig wiederhergestellt.

### Beispiel 5

Ein Absetzbecken mit 300 m² Fläche ist mit Erdölprodukten (harzige Stoffe, Asphaltene, Rohöl) mit einer Schichtdicke von 1,0 bis 1,5 cm verschmutzt (die Verschmutzung mit Erdölprodukten beträgt 558 kg). Es ist eine einmalige Behandlung mit der Paste der Biomasse der Bakterienkultur des Stammes Acinetobacter species (bicoccum) B-6445 durchgeführt worden. Die Paste enthält 2,5 kg absolut trockene Biomasse (Massenverhältnis der Biomasse zu den Erdölprodukten 1:10³), 5 kg Superphosphat und 3 kg Ammoniumsulfat. Nach 21 Tagen erfolgt eine vollständige (zu 100%) Reinigung des Behälters.

### Beispiele 6 - 11

Man reinigt 600 ml mit Rohöl verschmutztes Wasser. Das Wasser füllt man in 6 Kolben zu je 100 ml ab und setzt je 1 ml Rohöl hinzu. Die Reinigung nimmt man in den Kolben auf einer Schaukel vor. Dazu führt man in jeden Kolben Mineralsalze ein, die Stickstoff und Phosphor enthalten. Dann führt man in den ersten Kolben eine wässrige Suspension des Stammes Acinetobacter species (bicoccum) B-6445 (I), in den zweiten Kolben eine wässrige Suspension der Biomasse der Bakterienkultur des Stammes Rhodococcus species S-1213 (II), in den dritten Kolben eine wässrige Suspension der Biomasse der Bakterienkultur des Stammes Arthrobacter species S-1212 (III), in den vierten Kolben eine wässrige Suspension des Gemisches der Biomasse der Bakterienkultur der Stämme I und II, in den fünften Kolben eine wässrige Suspension der Biomasse der Bakterienkultur des Gemisches der Stämme I und III, in den sechsten Kolben eine wässrige Suspension der Biomasse der Bakterienkultur des Gemisches der Stämme I, II und III ein. Das Massenverhältnis der eingebrachten Einimpfungs-Biomasse zum Erdöl beträgt in allen Beispielen jeweils 1:10.

Nach 10 Stunden Mischzeit beträgt der Reinigungsgrad im ersten Kolben 15% im zweiten Kolben 13%, im dritten Kolben 12%, im vierten Kolben 70%, in fünften Kolben 65%, im sechsten Kolben 60% und im siebten Kolben 100%.

Nach 24 Stunden Mischzeit beträgt der Reinigungsgrad in allen Kolben 100%.

Die beanspruchten Stämme kennzeichnen sich durch eine kohlenwasserstoffoxydierende Aktivität und können zur Regenerierung der Ökologie der Umwelt in der erdölgewinnenden und erdölverarbeitenden Industrie und in anderen Industriezweigen benutzt werden. Das erfindungsgemässe Verfahren kann breite Anwendung finden in der Landwirtschaft bei der Rekultivierung von Böden, in der Wiederherstellung der Reinheit von Gewässern sowie in verschiedenen Bereichen der Volkswirtschaft, die mit der Gewinnung, Verarbeitung, Fortleitung und Verwendung von Erdöl und Erdölprodukten verbunden sind.

## Patentansprüche

1. Verfahren zur biologischen Reinigung von Ölverschmutzungen, das das Einbringen einer Bakterienkultur in die Verschmutzung einschließt, **dadurch gekennzeichnet,** daß man eine Bakterienkultur bestehend aus Acinetobacter species (bicoccum) B-6445, Arthrobacter species S-1212 und/oder Rhodococcus species S-1213, selektioniert aus aktiviertem Schlamm und hinterlegt am 19. Januar 1993 in der Unionskollektion industrieller Mikroorganismen des Unions-Forschungsinstituts für Genetik und Selektion von Mikroorganismen, in die Verschmutzung einbringt, wobei das Masseverhältnis der Biomasse der Bakterienkultur zur Ölverschmutzung jeweils 1:10 - 10⁵ beträgt.

2. Verfahren nach Anspruch 1, **dadurch gekennzeichnet,** daß man die biologische Reinigung in Anwesenheit von Mineralsalzen von Stickstoff und Phosphor durchführt.

3. Verfahren nach Anspruch 2, **dadurch gekennzeichnet,** daß man die biologische Reinigung in Anwesenheit von Eisen, Zink, Mangan und/oder Magnesium durchführt.

## Claims

1. Method of biologically cleaning oil pollution, which includes the introduction of a bacterial culture into the pollution, characterised in that a bacterial culture consisting of the Acinetobacter species (Bicoccum) B-6445, the Arthrobacter species S-1212 and/or the Rhodococcus species S-1213, selected from activated sludge and deposited on 19 January 1993 in the Unionskollektion industrieller Mikroorganismen des Unions-Forschungsinstituts für Genetik und Selektion von Mikroorganismen [union collection for industrial microorganisms at The Union-Research Institute for Genetics and Selection of Microorganisms], is introduced into the pollution, wherein the mass ratio of the biomass of the bacterial culture to oil pollution in each case amounts to 1:10 - 10⁵.

2. Method according to claim 1, characterised in that the biological cleaning process is carried out in the presence of mineral salts of nitrogen and phosphorous.

3. Method according to claim 2, characterised in that the biological cleaning process is carried out in the presence of iron, zinc, manganese and/or magnesium.

## Revendications

1. Procédé pour la purification biologique de pollutions par les hydrocarbures, qui comprend l'incorporation d'une culture bactérienne dans la pollution, caractérisé en ce qu'on incorpore dans la pollution une culture bactérienne constituée par Acinetobacter species (biococcum) B-6445, Arthrobacter species S-1212 et/ou Rhodococcus species S-1213 sélectionnées à partir de boues activées et déposées le 19 janvier 1993 dans la Collection de l'Union de Micro-organismes Industriels de l'Institut de Recherche de l'Union pour la Génétique et la Sélection de Micro-organismes, le rapport massique de la biomasse de la culture bactérienne à la pollution par les hydrocarbures s'élevant respectivement à 1:10 - 10⁵.

2. Procédé selon la revendication 1, caractérisé en ce qu'on effectue la purification biologique en présence de sels minéraux d'azote et de phosphore.

3. Procédé selon la revendication 2, caractérisé en ce qu'on effectue la purification biologique en présence de fer, de zinc, de manganèse et/ou de magnésium.
